# EUROPÄISCHE PATENTSCHRIFT

(11) **EP 1 381 351 B1**
(45) Veröffentlichungstag und Bekanntmachung des Hinweises auf die Patenterteilung: **13.10.2004**
(21) Anmeldenummer: 02722039.1
(22) Anmeldetag: 29.01.2002
(51) Int. Cl.: A61K 9/14, A61K 9/16, A61K 9/72, B01F 13/00, B01J 19/00, A61K 9/00, B01D 9/00, B01F 5/06

(54) **VERFAHREN ZUR KONTINUIERLICHEN HERSTELLUNG INHALIERFÄHIGER ARZNEISTOFFE**
METHOD FOR THE CONTINUOUS PRODUCTION OF MEDICAMENTS FOR INHALATION
PROCEDE DE PRODUCTION CONTINUE DE MEDICAMENTS A INHALER

(30) Priorität: 21.04.2001 DE 10119718
(43) Veröffentlichungstag der Anmeldung: 21.01.2004
(60) Teilanmeldung: 04017279.3
(73) Patentinhaber: Boehringer Ingelheim Pharma GmbH & Co.KG, 55218 Ingelheim am Rhein (DE)
(72) Erfinder: ZIERENBERG, Bernd, 55411 Bingen am Rhein (DE); SCHIEWE, Jörg, 55129 Mainz (DE)
(74) Vertreter: Fuchs Mehler Weiss & Fritzsche
(86) Internationale Anmeldenummer: PCT/EP2002/000885
(87) Internationale Veröffentlichungsnummer: WO 2002/085329

(56) Entgegenhaltungen:
- EP-A- 1 123 735
- WO-A-01/14036
- DE-A- 19 917 156
- US-A- 5 921 678

## Beschreibung

Die Erfindung betrifft ein Verfahren zur kontinuierlichen Herstellung inhalierfähiger Arzneistoffe.

Im Rahmen der Erfindung ist unter dem Begriff "Arzneistoff" der wirksame Bestandteil eines Arzneimittels zu verstehen, der üblicherweise auch als Pharmakon oder Wirkstoff bezeichnet wird.

Inhalativa erfordern eine bestimmte Erscheinungsform des Arzneistoffes. Zum Einsatz kommen in der Regel mikronisierte Arznei- bzw. Wirkstoffe in fester Form. Um die Inhalierfähigkeit des Arzneistoffes zu gewährleisten, werden hohe Anforderungen an die Teilchengröße, die Teilchengrößenverteilung, die Morphologie, die Stabilität und das Fließverhalten gestellt.

In der Regel gelangt nicht die gesamte inhalativ verabreichte Dosis des Arzneistoffes in die Lunge, sondern nur ein Teil dieser Dosis. Maßgeblichen Einfluß auf den Anteil des Arzneistoffes, der tatsächlich in die Lunge gelangt, hat die Teilchengröße. Aus diesem Grunde werden Teilchen bevorzugt, die einen Durchmesser kleiner 20µm, vorzugsweise kleiner 5µm und größer 0,3µm, aufweisen. Der Durchmesser des Teilchens sollte sich im angegebenen Fenster befinden und darüber hinaus eine möglichst enge Größenverteilung aufweisen. Größere Teilchen werden beim Einatmen bereits in den oberen Luftwegen abgeschieden, wohingegen kleinere Teilchen nicht in der Lunge deponiert werden und diese beim Ausatmen wieder verlassen.

Unter Teilchendurchmesser im Rahmen der vorliegenden Erfindung ist der aerodynamische Partikeldurchmesser zu verstehen, wobei dieser definiert ist als Äquivalentdurchmesser einer Kugel der Dichte von 1 g/cm³, die die gleiche Sentimedationsgeschwindigkeit in Luft besitzt, wie das untersuchte Teilchen.

Des weiteren werden hohe Anforderungen an die physikalische Stabilität der mikronisierten Arzneistoffteilchen gestellt. Die Teilchen sollten bei Umgebungsbedingungen vorzugsweise in der stabilen Kristallform vorliegen, um Agglomeration durch Phasenumwandung zu verhindern. Die Stabilität der Arzneistoffteilchen hat somit indirekten Einfluß auf die tatsächlich in die Lunge gelangte Arzneistoffmenge. Aus diesem Grunde werden hohe Anforderungen an die Stabilität des Arzneistoffes gestellt, um eine dauerhaft gleichbleibende Qualität, insbesondere eine zeitlich konstante Teilchengröße bzw. Größenverteilung, des Arzneistoffes zu gewährleisten. Gerade im Bereich der Pharmazie und der Verwendung von Arzneistoffen ist dieses Qualitätsmerkmal unerläßlich, weil die Wirkung des Arzneistoffes von der in die Lunge gelangten Dosis und somit, wie oben beschrieben, von der Teilchengröße und ihrer Größenverteilung abhängt.

Ähnliches gilt für die Morphologie der mikronisierten Teilchen, da die Beschaffenheit der Teilchenoberfläche direkten Einfluß auf die Neigung der Teilchen zur Agglomeration und somit indirekten Einfluß auf die Teilchengröße selbst bzw. die Haltbarkeit des Arzneistoffes hat.

Dem mikronisierten Arzneistoff können Hilfsstoffe zugesetzt werden, mit denen die physiko-chemischen Eigenschaften eines Arzneimittels eingestellt werden, wobei diese die qualitätsbestimmenden Parameter, wie Bioverfügbarkeit, Wirksamkeit und Haltbarkeit in gewünschter Weise beeinflussen.

Neben der Teilchengröße und Größenverteilung des mikronisierten Arzneistoffs können Art, Teilchengröße und Megenverhältnis der zugesetzten Hilfsstoffe in entscheidender Weise die Arzneistoff-Dosis beeinflussen, die in die Lunge gelangt.

Herkömmliche Verfahren zur Herstellung inhalierfähiger Arzneistoffe sind in der Regel, eine grob strukturierte Betrachtungsweise vorausgesetzt, zweistufig, wobei in einer ersten Stufe der Arzneistoff in fester, üblicherweise kristallinen Form hergestellt und dieser in einer zweiten Stufe im Rahmen eines Zerkleinerungsprozesses in mikronisierte Teilchen transformiert wird. Nach dem Stand der Technik kommen für den Zerkleinerungsprozeß Mahlprozesse zum Einsatz, wobei insbesondere Luftstrahlmahlen eine große Bedeutung erlangt hat, da es ökonomisch arbeitet, für eine Vielzahl von Substanzen anwendbar ist und eine einfache Abtrennung der gewünschten Teilchenfraktionen durch einen nachgeschalteten Zyklon-Abscheider erlaubt.

Nachteilig an dem nach dem Stand der Technik verwendeten Luftstrahlmahlen ist, daß die Feststoffteilchen prinzipbedingt einer erheblichen Krafteinwirkung während des Mahlprozesses ausgesetzt sind. Diese Krafteinwirkung induziert eine beträchtliche lokale Erwärmung und führt darüber hinaus zur Bildung amorpher Anteile. Aufgrund der lokalen Erwärmung eignet sich das Luftstrahlmahlen bzw. das Mahlen als Zerkleinerungsprozeß generell nicht für niedrigschmelzende, thermisch labile oder denaturierbare Stoffe.

Darüber hinaus wird bei der Lagerung strahlgemahlener Arzneistoffe häufig eine Agglomeration beobachtet, da die durch den Mahlprozeß entstandenen amorphen Anteile rekristallisieren.

Aus der WO 01/14036 ist ein Verfahren zu Herstellung eines inhalierfähigen Arzneistoffes bekannt. Hierbei wird eine Vorrichtung verwendet, in welcher zwei flüssige Ströme in vorgegebenen Volumenverhältnissen gezwungen werden, aufeinander zu stoßen.

Vor diesem Hintergrund ist es die Aufgabe der vorliegenden Erfindung ein Verfahren zur kontinuierlichen Herstellung inhalierfähiger Arzneistoffe bereitzustellen, bei dem die Einhaltung der genannten Anforderungen an Arzneistoffe sichergestellt ist und darüber hinaus die Nachteile der nach dem Stand der Technik verwendeten Verfahren vermieden werden.

Gelöst wird die Aufgabe durch ein kontinuierliches Verfahren zur Herstellung inhalierfähiger Arzneistoffe, welches folgende Verfahrensschritte umfaßt:
- Einsatz einer Arzneistoff-Lösung,
- Segmentierung der Lösung mittels eines Segmenters und eines Transportmediums,
- Einleiten und Führen des Kristallisationsprozesses in einer Verweilerstrecke mittels Aufprägen einer definierten Temperatur, wobei zunächst mittels einer sprunghaften Temperaturverringerung der Keimbildungsprozeß in der Art eingeleitet wird, daß die Lösung bzgl. der Temperatur T und der Konzentration C des in ihr gelösten Stoffes einen übersättigten, metastabilen oder labilen Zustand annimmt, und anschließend das Kristallwachstum durch gezielte Kühlung beinflußt wird, und
- Abscheiden der Kristallpartikel von den übrigen Phasen nach Durchlauf der Verweilerstrecke in einem Abscheider.

Erfindungsgemäß wird zunächst von einer Arzneistoff-Lösung ausgegangen. Es können bereits vorliegende Arzneistoff-Lösung verwendet werden, beispielsweise eine während der Arzneistoff-Herstellung ausfallende Lösung.

Diese Lösung wird im Anschluß einem Segmenter zugeführt und mit einem Transportmedium segmentiert. Bei dem Transportmedium handelt es sich um ein zweites, mit der Lösung nicht mischbares Fluid, mit dessen Hilfe die Lösung in Form diskreter Segmente in der Art unterteilt wird, daß die diskreten Segmente, welche vorzugsweise von gleichem Volumen sind, in einen aus dem Transportmedium bestehenden Trägerstom eingebracht werden. Das Transportmedium kann gasförmig oder flüssig sein, wobei sich in Versuchen gezeigt hat, daß mit Lösungsmitteldampf der Lösung gesättigte Luft besonders geeignet ist, um als Transportmedium zu dienen. Die beiden dem Segmenter zugeführten Ströme, daß heißt die Lösung einerseits und das Transportmedium andererseits, verlassen den Segmenter als segmentierte, vorzugsweise regelmäßig segmentierte Zweiphasenströmung.

Die Abmessungen der Segmenterkanäle des für das erfindungsgemäße Verfahren vorzugsweise zu verwendenden Segmenters liegen im Bereich von 0,1 mm bis 5 mm, vorzugsweise zwischen 0,2 und 1 mm. Es handelt sich dabei also um kleinste Strukturen.

Darüberhinaus bietet es sich an, den Segmenter konstruktiv so zu gestalten, daß er in der Temperatur leicht steuerbar ist, das heißt leicht beheizt/gekühlt werden kann und mit ihm große Abkühlraten - Temperaturdifferenz pro Zeiteinheit - zu erzielen sind. Dies wird durch Ausbildung kleinster Strukturen unterstützt.

Des weiteren können die Flußraten der Lösung und des Transportmediums in weiten Grenzen variiert werden, wodurch direkt Einfluß auf die Ausbildung und damit die Gestalt der segmentierten Zweiphasenströmung genommen werden kann. Vorzugsweise erfolgt die Einstellung so, daß etwa gleichgroße Fluidsegmente von einer Länge von zwei- bis dreimal dem Kanaldurchmesser erzeugt werden.

Nach der Segmentierung wird die segmentierte Zweiphasenströmung einer Verweilerstrecke zugeführt. Die Verweilerstrecke dient der eigentlichen Herstellung des inhalierfähigen Arzneistoffes bzw. der zu produzierenden Teilchen mit den genannten Eigenschaften bezüglich der Teilchengröße, der Größenverteilung der Teilchen, der Morphologie und der Stabilität.

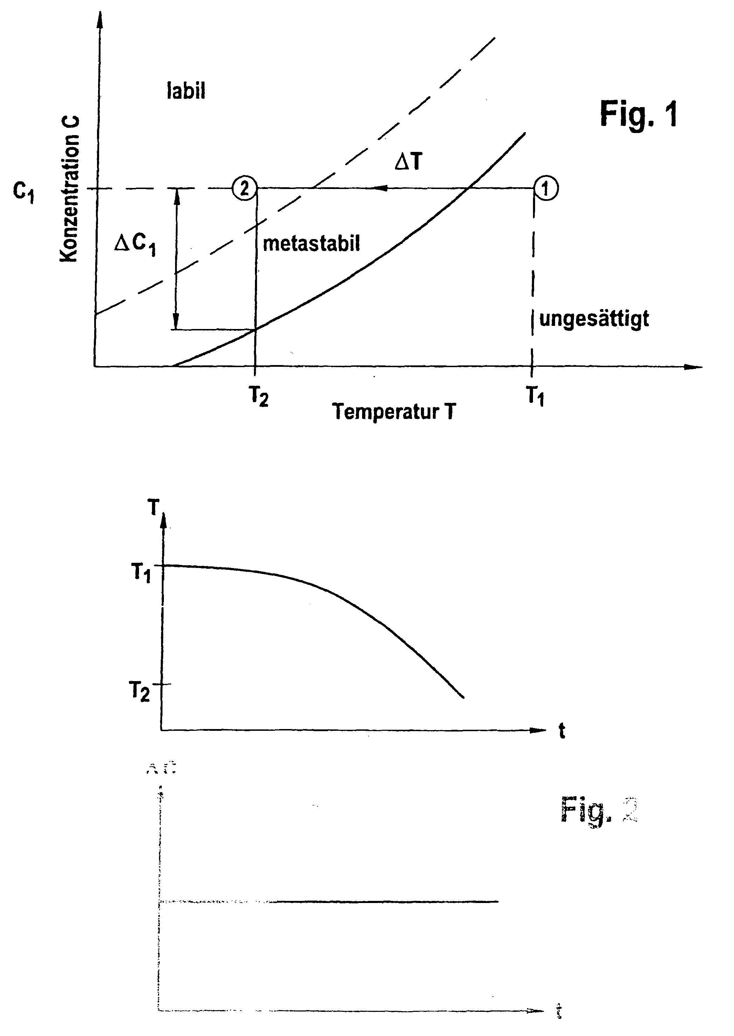

Bei Eintritt der Zweiphasenströmung in die Verweilerstrecke wird in einem ersten Schritt der Keimbildungsprozeß durch sprunghafte Temperaturverringerung eingeleitet. Zur näheren Erläuterung der in der Verweilerstrecke ablaufenden Vorgänge wird auf die Figur 1 verwiesen, die das Konzentrations-Temperatur-Diagramm der Lösung zeigt. In dem durch die y-Achse (Konzentration) und x-Achse (Temperatur) aufgespannten Quadranten sind zwei Kurven eingezeichnet, von denen eine durchgezogen und eine gestrichelt ist und durch die drei unterschiedliche Bereiche gebildet werden.

Der erste Bereich liegt rechts von der durchgezogenen Linie, wird also links von dieser begrenzt, und stellt den Bereich der ungesättigten Lösung dar, in welchem weder ein Keimbildungsprozeß einsetzt noch ein Kristallwachstum stattfindet. Ein Kristallwachstum, welches immer zunächst die Bildung eines Keimes voraussetzt, findet grundsätzlich erst in einer übersättigten Lösung statt, da bereits gebildete Kristalle bei Unterschreiten der Sättigungslinie (durchgezogene Linie) prinzipbedingt zumindest teilweise wieder in die Lösung übergehen.

Der links der durchgezogenen Linie liegende Bereich der übersättigten Lösung läßt sich wiederum in zwei Bereiche unterteilen, wobei der zwischen beiden Linien angesiedelte Bereich die metastabile Zone bildet, in der zwar ein Kristallwachstum aber kein Keimbildungsprozeß abläuft. Der dritte links von der gestrichelten Linie liegende Bereich bildet die labile Zone, in der als einzige der drei Bereiche ein spontaner Keimbildungsprozeß einsetzen kann.

Die Zweiphasenströmung bzw. die in ihr enthaltenen Segmente der Lösung weisen bei Eintritt in die Verweilerstrecke eine Konzentration C₁ auf, wobei die Lösung durch eine sprunghafte Temperaturverringerung von einem Zustand 1 in einen Zustand 2 überführt wird. Der Zustand 1 kann ein ungesättigter oder ein in der metastabilen Zone liegender Zustand sein, das heißt es kann bereits im Zustand 1 eine übersättigte Lösung vorliegen. Bei einem Mangel an Keimen ist es wesentlich für die Erfindung, daß der Zustand 2 in der labilen Zone liegt, was erfindungsgemäß durch eine entsprechend schlagartige Temperaturveränderung realisiert werden kann. Durch Überführen der Lösung in den labilen Zustand wird der Keimbildungsprozeß eingeleitet und der sich an ihn anschließende Kristallisationsprozeß wird durch Nachführen der Temperatur d.h. durch gezieltes Kühlen beeinflußt.

Ist ein Keimbildungsprozeß nicht erforderlich, weil genügend Kristallisationskeime vorliegen, ist die Durchführung des Verfahrens auch möglich, wenn Zustand 2 in der metastabilen Zone liegt.

Erst durch die beschriebenen Maßnahmen ist eine gezielte Einflußnahme auf das Kristallwachstum möglich. Dies trägt wesentlich zur Lösung der erfindungsgemäßen Aufgabe bei. Sehr kleine Partikel mit geringem Durchmesser im Bereich weniger Mikrometer erhält man in der labilen Zone, daß heißt bei sehr hoher Übersättigung, wenn sehr schnell sehr viele Keime gebildet werden, die kaum wachsen.

Aus diesem Grunde ist es erforderlich, die Verweilerstrecke für das erfindungsgemäße Verfahren konstruktiv so zu gestalten, daß sie in der Temperatur leicht steuerbar ist, das heißt leicht beheizt/gekühlt werden kann und mit ihr große Abkühlraten - Temperaturdifferenz pro Zeiteinheit - erzielbar sind. Daher eignen sich bei der Verweilerstrecke ebenfalls Strukturen im Bereich von einigen Millimetern bzw. Mikrometern.

Bei der Kristallisation nach dem Stand der Technik wird die beschriebene Vorgehensweise mit der sprunghaften Verlagerung der Zustandsgrößen vermieden, da durch die in kurzer Zeit frei werdende Kristallisationswärme Temperaturschwankungen in der Lösung hervorgerufen werden, die zu undefinierten Wachstumsbedingungen führen. Im Gegensatz hierzu kann bei dem erfindungsgemäßen Verfahren durch Verwendung von Apparaturen mit Dimensionen im Millimeter- oder Mikrometerbereich die Temperatur in der Lösung jederzeit exakt geregelt werden.

Daher weist die für das erfindungsgemäße Verfahren verwendete Verweilerstrecke wie bereits erwähnt kleine Strukturen auf, wobei ein möglichst hohes Oberflächen-/Volumenverhältnis angestrebt wird. Bei der Verweilerstrecke handelt es sich beispielsweise um eine schlauch-, rohr- oder kanalförmige Verweilerstrecke mit einem Durchmesser im Bereich von 0,5 bis 10 mm, vorzugsweise von 1 mm bis 2 mm, wobei je nach der Länge der Verweilerstrecke, die Ausmaße zwischen 10 cm bis 200 m, vorzugsweise zwischen 1 m bis 25 m, aufweisen kann, Verweilzeiten im Bereich von einigen Sekunden bis zu einigen Stunden realisiert werden können.

Der geringe Durchmesser der Verweilerstrecke bzw. das große Oberflächen-/Volumenverhältnis impliziert eine geringe in der Verweilerstrecke geführte Lösungsmenge, welche nur begrenzt Wärme speichern kann. Aus diesem Grunde und aufgrund des Oberflächen-Nolumenverhältnises kann der in der Verweilerstrecke befindlichen Lösung von außen in sehr kurzer Zeit eine definierte Temperatur aufgeprägt werden, wodurch eine schnelle Temperaturführung ermöglicht wird. Ebenfalls aufgrund der geringen Abmessungen werden nur sehr geringe Temperaturgradienten in der Lösung beobachtet und somit kann von einer weitestgehend homogenen Temperaturverteilung ausgegangen werden. Dies ist insofern hervorzuheben, da es für die Effektivität der Kristallisation wichtig ist, daß die lokalen Bedingungen in der Verweilerströmung nicht variieren und sich im gesamten Lösungsvolumen die gewünschten Parameter einstellen.

Entgegen den in dem Stand der Technik verwendeten Verfahren treten bei dem erfindungsgemäßen Verfahren keine unerwünschten Temperaturschwankungen auf, da die Temperatur in der Lösung von außen gezielt und schnell eingestellt werden kann und damit der Keimbildungsprozeß und das Kristallwachstum exakt gesteuert werden können. Würde die Lösung bei Verwendung von herkömmlichen Kristallisatoren entsprechend Figur 1 von Zustand 1 in Zustand 2 so überführt, daß eine hohe Übersättigung eintritt, käme es zu nicht beeinflußbaren Temperaturschwankungen, da der in Zustand 2 ablaufende Keimbildungsprozeß und die bei hoher Keimzahl entstehende, erhebliche Kristallisationswärme den Zustand hin zu höheren Temperaturen verschieben würde und ein Gegensteuern in Form eines Kühlens aufgrund der konstruktiven Abmessungen herkömmlicher Kristallisatoren zu periodischen Temperaturschwankungen führen würde.

Nach Durchlaufen der Verweilerstrecke wird das Produktgemisch einem Abscheider zugeführt, in welchem die erzeugten Kristallpartikel von den übrigen Phasen getrennt werden, so daß am Ende des Herstellungsverfahrens, nach Durchlaufen des Abscheiders der Arzneistoff mit den gewünschten Eigenschaften vorliegt.

Vorteilhaft sind Verfahren, bei denen die Arzneistoff-Lösung unter Auflösen des festen Arzneistoffes in einem Lösungsmittel zur Bildung einer solchen Arzneistoff-Lösung bereitgestellt wird.

Dabei wird der feste Arzneistoff in einem Lösungsmittel, in dem er sich bei vorgegebener Temperatur vollständig löst, zur Bildung einer Arzneistoff-Lösung aufgelöst.

Vorteilhaft sind Verfahren, bei denen
- die Segmentierung der Lösung mittels eines Segmenters und eines Transportmediums unter Ausbildung von Plug-Flow-Bedingungen erfolgt.

Wesentlicher Vorteil dieser Ausführungsform ist, daß die aus dem Segmenter austretende Zweiphasenströmung in der rohrförmigen Verweilerstrecke, die sie im Anschluß durchläuft, kein parabolisches Geschwindigkeitsprofil (Hagen-Poiseulliesches-Gesetz) ausbildet, sondern sich ein über die Zeit gemitteltes Rechteckprofil einstellt. Dies unterscheidet das erfindungsgemäße Verfahren von den in herkömmliche Kristallisatoren ablaufenden Verfahren, bei denen sich ein parabolisches Profil einstellt, so daß sich die Geschwindigkeit in den Rohrrandbereichen verringert und schließlich direkt an der Wand zu Null wird.

Als Folge dieses parabolischen Geschwindigkeitsprofils kommt es an der Rohrinnenwand der herkömmlichen Kristallisatoren zu Ablagerungen insbesondere von weiter wachsenden Kristallen, wodurch die Kristallisatoren letztendlich verstopfen bzw. sich zu setzen.

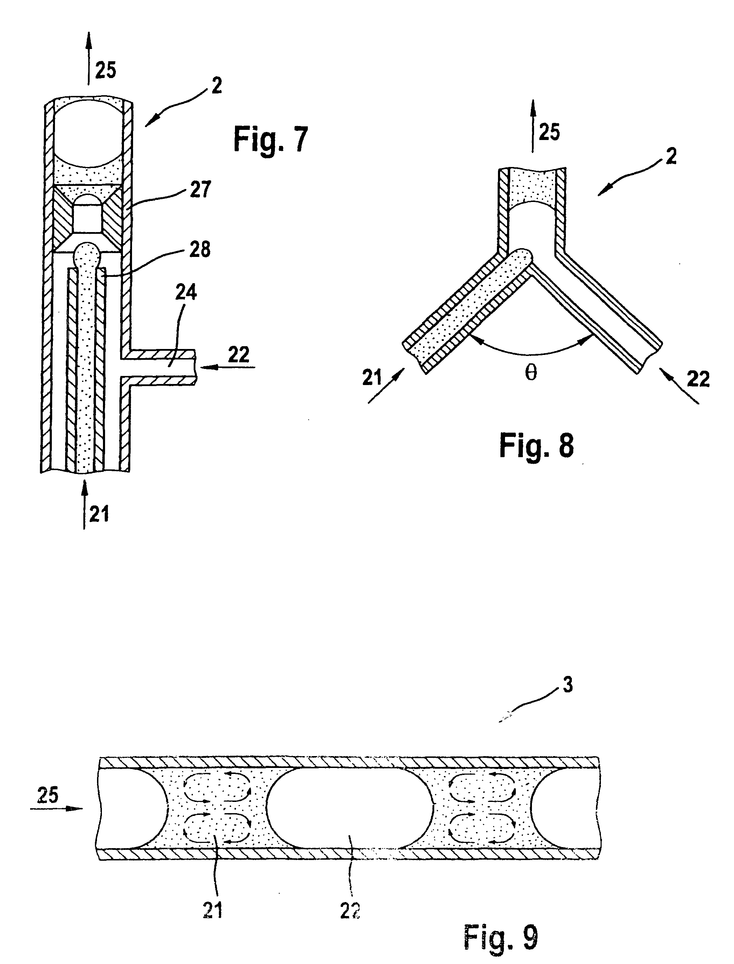

Bezüglich der Plug-Flow-Bedingungen sei auf Figur 9 verwiesen, in dem die in den Volumensegmenten vorliegenden Strömungen bzw.

Strömungsrichtungen eingezeichnet sind. Die Strömungsvorgänge in den Segmenten unterstützen die Homogenisierung der Lösung und wirken der Ausbildung von Konzentrationsunterschieden im Inneren und an der Wand entgegen. Des weiteren wird ein Verstopfen durch Kristall-Ablagerung an den Innenwänden der Verweilerstrecke vermieden.

Vorteilhaft sind Ausführungsformen des Verfahrens, die dadurch gekennzeichnet sind, daß
- die Arzneistoff-Lösung in einem Mischer mit einem Fällungsmittel zu einer homogenen Fällungslösung vermengt wird und diese Fällungslösung das weitere Verfahren durchläuft.

Das Fällungsmittel wird der Arzneistoff-Lösung zugemischt, um die Löslichkeit des Arzneistoffs in der Mischung bei gegebener Tempertur zu vermindern, so daß Festkörperpartikel gebildet werden. Diese Arnzeistoff-Lösung wird dabei in einem dem Segmenter vorgeschalteten Verfahrensschritt zur Herstellung einer möglichst homogenen Fällungslösung mit einem Fällungsmittel in einem Mischer durchmengt.

Mikromischer eignen sich hierzu besonders gut, da in ihnen Masse- und Wärmetransportvorgänge schnell und effizient ablaufen. Unter Mikromischer im Rahmen der vorliegenden Erfindung ist eine Struktur zu verstehen, welche Dimensionen im Bereich von 10µm bis 1 mm, vorzugsweise zwischen 25µm bis 200µm, aufweist.

Zur Herstellung von mikronisierten und inhalierfähigen Arzneistoffen ist eine Vermischung der Arzneistoff-Lösung und dem Fällungsmittel zu einer Fällungslösung von möglichst hoher Homogenität erforderlich. Ein Mikromischer mit seinen filigranen Strukturen eignet sich hierfür in besonderem Maße. Bei ihm werden die beiden Fluide, einerseits die Arzneistoff-Lösung und anderseits das Fällungsmittel, nach Eintritt in den Mischer mittels einer Mikrostruktur in Einzelströme aufgeteilt. Die Einzelströme sind beispielsweise lamellenförmig und werden mit Hilfe von in der Mikrostruktur angeordneten Kanälen in der Art geschichtet, daß ein System aus dünnen Fluidlamellen entsteht, bei welchem abwechselnd eine Fluidlamelle der Arzneistoff-Lösung einer Fiuidlamelle des Fällungsmittels benachbart ist. Dabei beträgt die Lamellendichte im Mikromischer 10 bis 1000, vorzugsweise 20 bis 500 pro cm. Das so geschichtete, aus einer Vielzahl von Lamellen bestehende Fluidsystem wird einer Mischkammer zugeführt, in der eine Vermischung durch Diffusion erfolgt. Die Mischung nach dem Prinzip der Diffusion kann nur in akzeptabel kurzen Zeiten vollzogen werden, wenn die Strukturen des Mischers und damit die Lamellendicke der Einzelströme genügend klein sind (vorzugsweise 10 bis 200 µm).

Die Dicke der Fluidlamellen bestimmt in entscheidender Weise die Zeit, die zum Ausgleich der Konzentrationsunterschiede durch diffusive Vermischung benötigt wird. Liegt die Lamellendicke im Bereich einiger zehn Mikrometer, kann eine komplette Vermischung und damit eine Homogenität der Fällungslösung im gesamten Mischvolumen bereits in einer Zeit unter einer Sekunde realisiert werden.

Der Mikromischer ist vorzugsweise in der Art ausgeführt, daß er auf einfache Weise beheizt und/oder gekühlt werden kann. Durchflußgeschwindigkeit und Temperatur im Mikromischer werden im Hinblick auf den Keimbildungsprozeß so gewählt, daß eine Keimbildung im Mischer nicht stattfindet.

An dieser Stelle sei darauf hingewiesen, daß eine Vielzahl von Vorrichtungen zum Heizen und Kühlen der einzelnen, beschriebenen Bauelemente des Mikroreaktors verwendet werden können. Insbesondere sind dies Draht-Widerstandsheizungen, elektrische Heizfolien, Peltier-Elemente sowie Heiz- und/oder Kühlvorrichtungen, die mit einem temperierten Fluid wie beispielsweise Wasser, Öl, Luft, Stickstoff und dergl. arbeiten. Daneben können auch Infrarot-Strahlung und Mikrowellenheizungen eingesetzt werden.

Vorteilhaft sind auch Ausführungsformen des Mikroreaktors, bei denen auf der Basis einer geätzten Platte, die eine elektrische Heizung aufweist, ein Mikroreaktor in diese Platte implementiert ist.

Vorteilhaft sind Ausführungsformen des Verfahrens, bei denen submikrometergroße Festkörperpartikel zur Keimbildung im Segmenter eingetragen werden, wobei vorzugsweise
- der Eintrag der submikrometergroßen Festkörperpartikel bei der Verwendung von Luft als Transportmedium vorzugsweise durch Zugabe eines Hilfsstoffes oder des Arzneisstoffes als Staubaerosol in den Luftstrom erfolgt, und
- bei Verwendung eines flüssigen Transportmediums submikrometergroße Festkörperpartikel in Form von Hilfsstoff- oder Arzneistoffpartikeln dem Transportmedium zugesetzt werden, oder
- bei Verwendung eines flüssigen Transportmediums submikrometergroße Festkörperpartikel in Form von Hilfsstoff-Kolloiden dem Transportmedium zugesetzt werden.

Durch Eintrag von submikrometergroßen Festkörperpartikeln läßt sich der Kristallisierungsprozeß besser kontrollieren. Durch den Eintrag von Kristallkeimen kann der Arbeitsbereich auch auf den metastabilen Bereich (bei geringeren Übersättigungsverhältnissen) erweitert werden. Bei den drei genannten Varianten des Feststoffpartikeleintrages werden bei der im Segmenter erzeugten Zweiphasen- strömung als Kristallisationskeime dienende Feststoffpartikel von außen, d.h. vom Transportmedium, in die segmentierte Lösung eingetragen.

Vorteilhaft sind Ausführungsformen des Verfahrens, bei dem submikrometergroße Festkörperpartikel zur Keimbildung im Mischer zugegeben werden, wobei vorzugsweise die Zugabe der submikrometergroßen Festkörperpartikel durch Verwendung eines Fällungsmittels, welches Kolloidpartikel enthält, erfolgt.

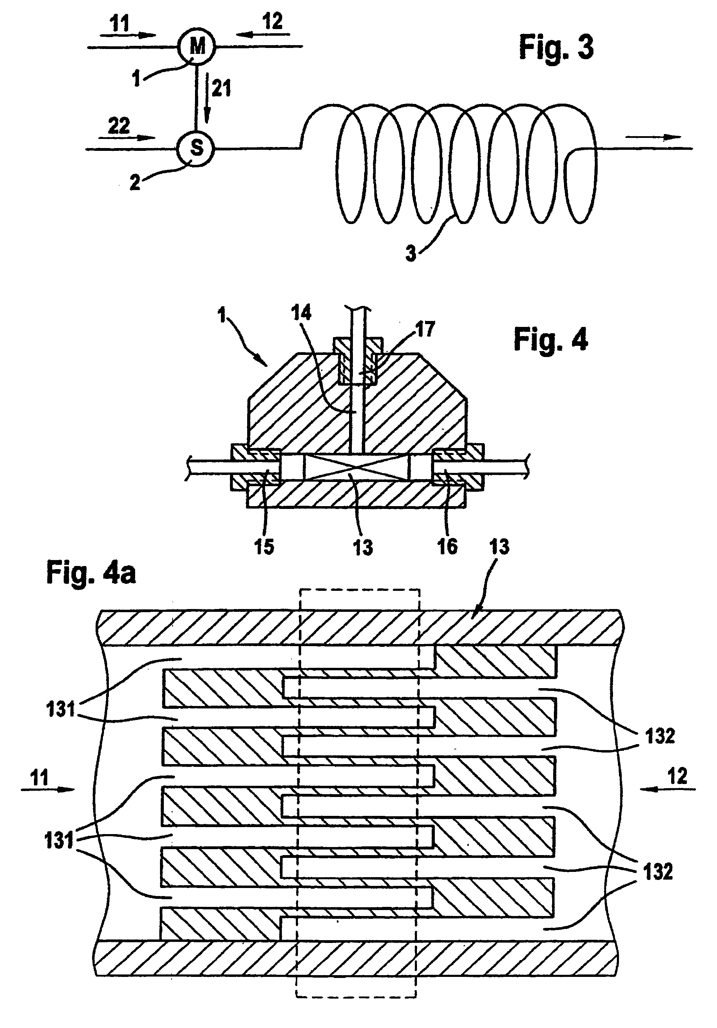

Vorteilhaft sind Ausführungsformen des Verfahrens, bei denen die Temperaturführung in der Verweilerstrecke in der Art erfolgt, daß eine im wesentlichen konstante Übersättigung C₁ in der Lösung vorliegt. Dies trägt zur Lösung der erfindungsgemäßen Aufgabe bei, sehr kleine Teilchen bzw. Kristalle von geringem Durchmeser zu bilden. Figur 2 zeigt wie die Temperatur T(t) zu führen ist, um bei einem parabolischen Verlauf der Konzentration (Figur 1) und unter der Annahme eines diffusionskontrollierten Kristallwachstums eine konstante Übersättigung über der Zeit zu erhalten (ebenfalls Figur 2).

Die Erfindung wird anhand verschiedener Ausführungsbeispiele gemäß den Zeichnungsfiguren näher erläutert. Hierbei zeigt:
- Figur 1: das Konzentrations-Temperatur-Diagramm der in die Verweilerstrecke eintretenden Lösung,
- Figur 2: die Temperaturführung T(t) einer Ausführungsform des erfindungsgemäßen Verfahrens zur Einstellung einer konstanten Übersättigung C (t),
- Figur 3: schematisch eine Ausführungsform eines Mikroreaktors zur Durchführung des erfindungsgemäßen Verfahrens,
- Figur 4: einen Mikromischer des Mikroreaktors gemäß einer ersten Ausführungsform im Querschnitt,
- Figur 4a: eine Vergrößerung der Strukturen der Kanäle des in Figur 4 dargestellten Mikromischers,
- Figur 5: einen Segmenter des Mikroreaktors gemäß einer ersten Ausführungsform im Querschnitt,
- Figur 6: einen Mikromischer des Mikroreaktors gemäß einer zweiten Ausführungsform mit integriertem Segmenter im Querschnitt,
- Figur 7: einen Segmenter des Mikroreaktors gemäß einer dritten Ausführungsform im Querschnitt,
- Figur 8: einen Segmenter des Mikroreaktors gemäß einer vierten Ausführungsform im Querschnitt,
- Figur 9: die Verweilerstrecke einer Ausführungsform eines Mikroreaktors im Querschnitt mit der in der Verweilerstrecke geführten Zweiphasenströmung, und
- Figur 10: eine Verweilerstrecke einer Ausführungsform eines Mikroreaktors mit den Temperaturverläufen über die Länge der Verweilerstrecke und über die Zeit.

Die Figuren 1 und 2 wurden bereits oben beschrieben.

Figur 3 zeigt schematisch den Aufbau eines Mikroreaktors zur Durchführung einer ersten Variante des Verfahrens. Der Mikroreaktor besteht aus einem Mikromischer 1, einem Segmenter 2 und einer Verweilerstrecke 3. Dabei wird zunächst die Arzneistoff-Lösung 11 mit einem Fällungsmittel 12 im Mikromischer 1 zu einer möglichst homogenen Fällungslösung 21 vermischt. Diese Fällungslösung 21 wird dem Segmenter 2 zugeführt und mit Hilfe eines Transportmediums 22 segmentiert. Die nach Durchlaufen des Segmenters vorliegende Zweiphasenströmung wird der Verweilerstrecke 3 zugeführt, in der die Kristallisation erfolgt.

Figur 4 zeigt den prinzipiellen Aufbau eines Mikromischers 1 mit den Eintrittsöffnungen 15 und 16 für die Zuführung der Arzneistoff-Lösung 11 einerseits und dem Fällungsmittel 12 andererseits, sowie der Austrittsöffnung 17, durch die die im Mikromischer 1 erzeugte Fällungslösung 21 den Mikromischer verläßt. Zwischen den Eintrittsöffnungen 15 und 16 ist die Mikrostruktur 13 angeordnet, mit der die eintretenden Fluidströme 11 und 12 in Einzelströme aufgeteilt werden. Die eigentliche Mischkammer 14 ist oberhalb dieser Mikrostruktur 13 angeordnet. In dieser Mischkammer 14 erfolgt die Durchmischung zu einer homogenen Lösung infolge Diffusion.

In Figur 4a dargestellt ist eine Vergrößerung der zwischen den Eintrittsöffnungen 15 und 16 liegenden Mikrostruktur 13. Die Zuführkanäle 131 und 132 zur Zuführung der Fluidströme in die Mischkammer 14 und zur Unterteilung der eintretenden Fluidströme in Teilströme sind in der Art angeordnet, daß die durch sie aufgetrennten Teilströme ein alternierendes System aus dünnen Fluidlamellen bilden, wobei alternierend ausdrückt, daß Fluidlamellen der Arzneistofflösung 11 und Fluidlamellen des Fällungsmittels 12 abwechselnd geschichtet werden.

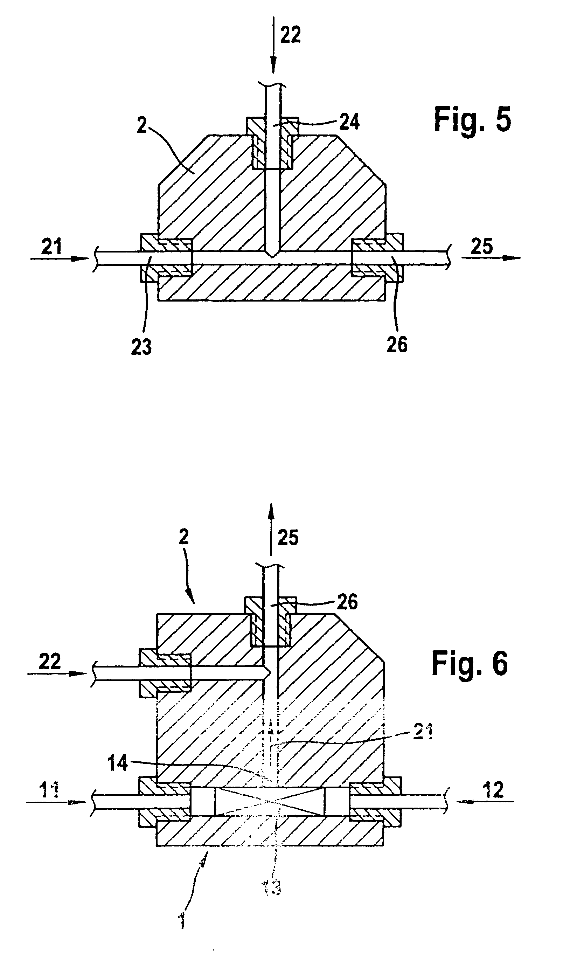

Figur 5 zeigt schematisch den Aufbau eines Segmenters 2 mit den Eintrittsöffnungen 23 und 24 und der Austrittsöffnung 26. Die durch die Eintrittsöffnung 23 dem Segmenter 2 zugeführte Fällungslösung 21 wird mit Hilfe des durch die Eintrittsöffnung 24 zugeführten Transportmediums 22 segmentiert und verläßt den Segmenter 2 als Zweiphasenströmung 25.

Figur 6 zeigt ein zweites Ausführungsbeispiel eines Mikromischers 1 mit einem integrierten Segmenter 2. Die in den Mikromischer 1 eintretenden Fluidströme, die Arzneistoff-Lösung 11 einerseits und das Fällungsmittel 12 andererseits gelangen durch die Mikrostruktur 13 des Mikromischers 1 in die Mischkammer 14 und werden nach Durchmischung in der Mischkammer 14 als weitestgehend homogene Fällungslösung 21 dem Segmenter 2 zugeführt. Im Segmenter 2 wird die Fällungslösung 21 mit einem zweiten, mit der Fällungslösung 21 nicht mischbaren Fluid, das als Trägermedium 22 dient, segmentiert, wobei die dadurch erzeugte Zweiphasenströmung 25 den Segmenter 2 durch die Austrittsöffnung 26 verläßt.

Figur 7 zeigt einen Segmenter 2, der zwei Rohre 27,28 umfaßt, von denen das kleinere Rohr 28 in dem größeren Rohr 27 und zu diesem koaxial angeordnet ist. Dabei wird die Fällungslösung 21 mittels des kleinen Rohres 28 in das große Rohr 27 eingeleitet und durch das seitliche durch die Eintrittsöffnung 24 zugeführte Transportmedium 22 segmentiert, so daß eine Zweiphasenströmung 25 den Segmenter 2 am Ende des großes Rohres 27 verläßt.

Figur 8 zeigt ein weiteres Ausführungsbeispiel eines Segmenters 2, bei dem die Zuführkanäle, die die Fällungslösung 21 einerseits und das Transportmedium 22 andererseits zuführen, einen Winkel bilden, der zwischen 0 und 180° variieren kann und bei dem in Figur 8 gezeigten Ausführungsbeispiel 90° beträgt.

Figur 9 zeigt einen Ausschnitt einer rohrförmigen Verweilerstrecke 3 im Querschnitt. Dargestellt ist der Aufbau der der Verweilerstrecke 3 zugeführten Zweiphasenströmung 25. Diese besteht aus vorzugsweise gleichgroßen Fluidvolumen von Fällungslösung 21, die mit Hilfe des Transportmediums 22 segmentiert sind.

Ebenfalls in Figur 9 dargestellt sind die in den Segmenten der Fällungslösung 21 ablaufenden Strömungsvorgänge bzw. die dazugehörigen Strömungsrichtungen. Die über die Zeit gemittelten Geschwindigkeiten ergeben einen über den Rohrquerschnitt der Verweilerstrecke 3 gleichmäßiges Rechteckprofil. Ersichtlich wird, daß sich unter den im Fluidsegment herrschenden Strömungsbedingungen keine Ablagerungen an den Rohrinnenwänden der Verweilerstrecke 3 bilden können.

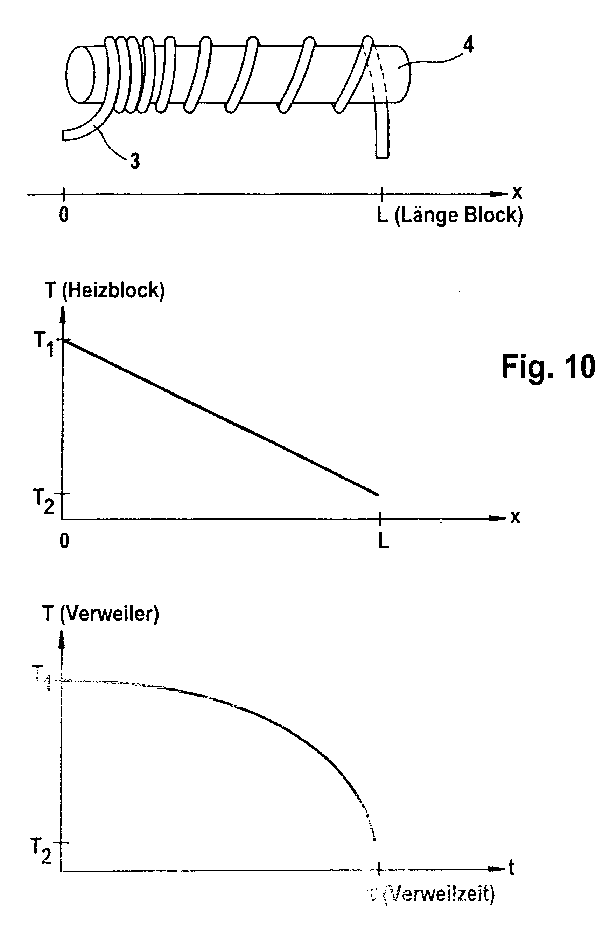

Figur 10 zeigt ein Ausführungsbeispiel einer Verweilerstrecke in der perspektivischen Ansicht, bei der eine schlauchförmige Verweilerstrecke 3 auf ein zylinderförmiges Aluminiumprofil 4 gewickelt ist. Die Verweilerstrecke 3 kann über das Aluminiumprofil 4 gekühlt bzw. geheizt werden.

Wie bereits erwähnt ist es eine Ausführungsform des Verfahrens, in der Verweilerstrecke 3 eine im wesentlichen konstante Übersättigung C₁ (siehe Figur 2 unten) in der Lösung zu realisieren. Dies wird mit einem Temperaturprofil erreicht, bei dem der Temperaturgradient mit der Zeit betragsmäßig zunimmt (siehe Figur 2 oben und Figur 10 unten).

Dieser Temperaturverlauf T(t) wird einerseits durch die Steigung der Verweilerstrecke 3 auf dem Aluminiumblock 4 (siehe Figur 10 oben) und andererseits durch das Temperaturprofil T(L) im Aluminiumblock 4 (siehe Figur 10 Mitte) realisiert, wobei im vorliegenden Beispiel die Temperatur im Aluminiumblock 4 linear abnimmt und die Steigung der auf den Aluminiumblock aufgewickelten Verweilerstrecke 3 zunimmt.

Die Steigung der aufgewickelten Verweilerstrecke 3 und der Temperaturverlauf T(L) im Aluminiumblock 4 sind dem jeweiligen Einzelfall anzupassen. Sie sind abhängig von dem verwendeten Arzneistoff, der Lösung, dem zusätzlichen Eintrag von Festkörperpartikeln als Kristallkeime und dem eventuell eingesetzten Fällungsmittel.

Im folgenden werden für die Wirkstoffe, die Hilfsstoffe, die Lösungs- und Fällungsmittel Beispiele angeführt.

Als Arzneistoffe bzw. Wirkstoffe werden eingesetzt:
- als Anticholinergika: Ipratropiumbromid, Tiotropiumbromid, Tiotropiumbromid-Monohydrat,
- als Betasympathomimetica: Bambuterol, Bitolterol, Carbuterol, Formoterol, Clenbuterol, Fenoterol, Hexoprenalin, Procaterol, Ibuterol, Pirbuterol, Tulobuterol, Reproteroi, Salbutamol, Sulfonterol, Terbutalin, Orciprenalin, 1-(2-Fluor-4-hydroxyphenyl)-2-[4-( 1-benzimidazolyi)-2-methyl-2-butylamino]ethanol,erythro-5'-Hydroxy-8'-(1-hydroxy-2-isopropylaminobutyl)-2H-1,4-benzoxazin-3-(4H)-on, 1-(4-Amino-3-chlor-5-trifluormethylphenyl)-2-tert.-butyl-amino)ethanol,1-(4-Ethoxycarbonylamino-3-cyan-5-fluorphenyl)-2-(tert.butylamino)ethanol,
- als Antiallergika: Dinatriumcromglicat, Nedocromil, Epinastin, und
- als Steroide: Flunisolid, Dexamethason-21-isonicotinat, Seratrodast, Mycophenolate mofetil, Pranlukast, Zileuton, Butixocort, Budesonid, Deflazacort, Fluticason, Proedrol, Mometasin furoat, Tipredan, Beclometason (bzw. das17, 21-Dipropionat), Beclomethason, Douglas, Icomethason enbutat, Ciclometason, Cloprednol, Fluocortin butyl, Halometason, Deflazacort, Alclometason, Ciclometason, Alisactid, Prednicarbat, Hydrocortison-butyratpropionat, Tixocortol-pivalat, Alclometason-dipropionat, Lotrison, Canesten-HC, Deprodon, Fluticason-propionat, Methylprednisolon-Aceponat, Halopredonacetat, Mometason, Mometasone-furoat, Hydrcortison-aceponat, Mometason, Ulobetasol-propionat, Aminogluethimid, Triamciolon, Hydrcortison, Meprednison, Fluorometholon, Dexamethason, Betamethason, Medryson, Fluclorolon acetonid, Fluocinolon acetonid, Paramethason-acetat, Deprodon Propionat, Aristocort-diacetat, Fluocinonid, Mazipredon, Difluprednat, Betamethason valerat, Dexamethasonisonicotinat, Beclomethason-Dipropionat, Fluocortoloncapronat, Formocortal, Triamcinolon-Hexacetonid, Cloprednol, Formebolon, Clobetason, Endrisone, Flunisolid, Halcinonid, Fluazacort, Clobetasol, Hydrocortison-17-Butyrat, Diflorason, Fluocortin, Amcinonid, Netamethason Diprpionat, Cortivazol, Betamethasonadamantoat, Fluodexan, Trilostan, Budesonid, Clobetason, Demetex, Trimacinolon Benetonid, 9.alpha.-chloro-6.alpha.-fluoro-11.beta.17.alpha.-dihydroxy-16.alpha.-methyl-3-oxo-1,4-androstadien-17.beta.-carboxysäure-methylester-17-propionat.

Sonstige mit dem erfindungsgemäßen Verfahren hergestellte Arzneistoffe sind Montelukast und Pramipexol.

Als Hilfsstoffe werden für Inhalativa insbesonders Lactose, Glucose, Sucrose, Mannitol, und/oder Trehalose verwendet.

Beispiele für Lösungs- und Fällungsmittel in Abhängigkeit von den herzustellenden Wirkstoffen zeigen die folgenden Tabellen, wobei Lösungs- und Fällungsmittel mischbar sein müssen.

### Für Anticholinergikal/Betasympathomimetica/Antiallergika:

| Wirkstoffe | Lösungsmittel | Fällungsmittel |
|---|---|---|
| Salzformen | Wasser, Methanol | Alkohole (Ethanol, Propanol, iso-Propanol), Ketone (Aceton, Butanon) |
| Freie Basen | Alkohole (Ethanol, Propanol, iso-Propanol, tert.-Butanol), Ketone (Aceton, Butanon) | Wasser, Methanol |

### Für Steroide:

| Wirkstoffe | Lösungsmittel | Fällungsmittel |
|---|---|---|
| Polare | Ketone (Aceton, Butanon) | Alkohole (Methanol, Ethanol) |
| | Alkohole (Ethanol, Propanol, iso-Propanol, tert.-Butanol), Ketone (Aceton, Butanon) | Wasser, Methanol |
| | Aromaten (Toluol, Ethylbenzol) | Alkohole (Ethanol, Propanol, iso-Propanol) |
| Unpolare | Halogenkohlenwasserstoffe (Dichlormethan, Trichlormethan | Alkohole (Ethanol, Propanol, iso-Propanol), Ether (Dimethylether, Dioxan) |

Beispiele für Transportmedien in Abhängigkeit von den herzustellenden Wirkstoffen und den verwendeten Lösungsmitteln zeigen die folgenden Tabellen, wobei Lösungsmittel und Transportmedien nicht mischbar sind.

| Wirkstoffe | Lösungsmittel | Transportmedien |
|---|---|---|
| Polare | Wasser, Alkohole (Methanol, Ethanol, Propanol, iso-Propanol, tert.-Butanol), Ketone (Aceton, Butanon) | Flüssigkeiten: Kohlenwasserstoffe (Benzine, Petrolether, Cyclohexan, Decalin, Benzol, Toluol, Xylole) Gase: Luft, Stickstoff, Kohlendioxid, Helium, Argon |
| Unpolare | Halogenkohlenwasserstoffe (Dichlormethan, Trichlormethan), Ether (Diethylether, Dibutylether), Aromaten (Toluol, Ethylbenzol) | Flüssigkeiten Wasser, Alkohole (Methanol), Amide (Formamid) Gase: Luft, Stickstoff, Kohlendioxid, Helium, Argon |

### Bezugszeichenliste

- 1: Mikromischer
- 2: Segmenter
- 3: Verweilerstrecke
- 4: Aluminiumprofil
- 11: Arzneistoff-Lösung
- 12: Fällungsmittel
- 13: Mikrostruktur
- 14: Mischkammer
- 15: Eintrittsöffnung
- 16: Eintrittsöffnung
- 17: Austrittsöffnung
- 21: Fällungslösung
- 22: Transportmedium
- 23: Eintrittsöffnung
- 24: Eintrittsöffnung
- 25: Zweiphasenströmung
- 26: Austrittsöffnung
- 27: Großes Rohr
- 28: Kleines Rohr
- 131: Zuführungskanäle
- 132: Zuführungskanäle

## Patentansprüche

1. Verfahren zur kontinuierlichen Herstellung inhalierfähiger Arzneistoffe, welches folgende Verfahrensschritte umfaßt:
- Einsatz einer Arzneistoff-Lösung (11),
- Segmentierung der Lösung mittels eines Segmenters und eines Transportmediums (22),
- Einleiten und Führen des Kristallisationsprozesses in einer Verweilerstrecke (3) mittels Aufprägen einer definierten Temperatur, wobei zunächst mittels einer sprunghaften Temperaturverringerung der Keimbildungsprozeß in der Art eingeleitet wird, daß die Lösung bzgl. der Temperatur T und der Konzentration C des in ihr gelösten Stoffes einen übersättigten, metastabilen oder labilen Zustand annimmt, und anschließend das Kristallwachstum durch gezielte Kühlung beinflußt wird, und
- Abscheiden der Kristallpartikel von den übrigen Phasen nach Durchlauf der Verweilerstrecke in einem Abscheider. .

2. Verfahren nach Anspruch 1, **dadurch gekennzeichnet, daß** die Arzneistoff-Lösung (11) unter Auflösen des festen Arzneistoffes in einem Lösungsmittel zur Bildung einer solchen Arzneistoff-Lösung (11) bereitgestellt wird.

3. Verfahren nach Anspruch 1 oder 2, **dadurch gekennzeichnet, daß**
- die Segmentierung der Lösung mittels eines Segmenters und eines Transportmediums (22) unter Ausbildung von Plug-Flow-Bedingungen erfolgt.

4. Verfahren nach einem der vorherigen Ansprüche, **dadurch gekennzeichnet, daß**
- die Arzneistoff-Lösung (11) in einem Mischer mit einem Fällungsmittel (12) zu einer homogenen Fällungslösung (21) vermengt wird und diese Fällungslösung das weitere Verfahren durchläuft.

5. Verfahren nach einem der vorherigen Ansprüche, **dadurch gekennzeichnet, daß** submikrometergroße Festkörperpartikel zur Keimbildung im Segmenter eingetragen werden.

6. Verfahren nach Anspruch 5, **dadurch gekennzeichnet, daß** der Eintrag der submikrometergroßen Festkörperpartikel bei der Verwendung von Luft als Transportmedium (22) durch Zugabe des Arzneisstoffes als Staubaerosol oder durch Zugabe eines Hilfsstoffes als Staubaerosol in den Luftstrom erfolgt.

7. Verfahren nach Anspruch 5, **dadurch gekennzeichnet, daß** bei Verwendung eines flüssigen Transportmediums (22) submikrometergroße Festkörperpartikel in Form von Hilfstoff- oder Arzneistoffpartikeln dem Transportmedium zugesetzt werden.

8. Verfahren nach Anspruch 5, **dadurch gekennzeichnet, daß** bei Verwendung eines flüssigen Transportmediums (22) submikrometergroße Festkörperpartikel in Form von Hilfsstoff-Kolloiden dem Transportmedium zugesetzt werden.

9. Verfahren nach Anspruch 4, **dadurch gekennzeichnet, daß** submikrometergroße Festkörperpartikel zur Keimbildung im Segmenter zugegeben werden.

10. Verfahren nach Anspruch 9, **dadurch gekennzeichnet, daß** die Zugabe der submikrometergroßen Festkörperpartikel durch Verwendung eines Fällungsmittels (12), welches Kolloidpartikel enthält, erfolgt.

11. Verfahren nach einem der vorherigen Ansprüche, **dadurch gekennzeichnet, daß** die Temperaturführung in der Verweilerstrecke in der Art erfolgt, daß eine im wesentlichen konstante Übersättigung in der Lösung vorliegt.

## Claims

1. Process for the continuous production of inhalable medicaments, which comprises the following process steps:
- use of a medicament solution (11),
- segmenting the solution by means of a segmenter and a transport medium (22),
- induction and guidance of the crystallisation process in a retention stretch (3) by subjecting it to a defined temperature, wherein the seed formation process is first induced by means of a rapid temperature reduction in such a way that the solution adopts a supersaturated, metastable or unstable state with regard to the temperature T and the concentration C of the material dissolved therein, and then the crystal growth is influenced by controlled cooling, and
- separating off the crystal particles from the other phases in a separator after passing through the retention stretch.

2. A process according to claim 1, **characterised in that** the medicament solution (11) is prepared by dissolving the solid medicament in a solvent to form such a medicament solution (11).

3. A process according to claim 1 or 2, **characterised in that**
- segmenting of the solution is carried out by means of a segmenter and a transport medium (22) with the formation of plug-flow conditions.

4. A process according to one of the preceding claims, **characterised in that**
- the medicament solution (11) is mixed in a mixer with a precipitation agent (12) to form a homogenous precipitation solution (21) and that this precipitation solution passes through the further process.

5. A process according to one of the preceding claims, **characterised in that** sub-micron-sized solid particles are added in the segmenter to form seeds.

6. A process according to claim 5, **characterised in that**, when air is used as the transport medium (22), the sub-micron-sized solid particles are introduced by adding the medicament as a dust aerosol or by adding an adjuvant as a dust aerosol to the air stream.

7. A process according to claim 5, **characterised in that**, when a fluid transport medium (22) is used, sub-micron-sized solid particles in the form of adjuvant or medicament particles are added to the transport medium.

8. A process according to claim 5, **characterised in that**, when a fluid transport medium (22) is used, sub-micron-sized solid particles in the form of adjuvant colloids are added to the transport medium.

9. A process according to claim 4, **characterised in that** sub-micron-sized solid particles are added in the segmenter to form seeds.

10. A process according to claim 9, **characterised in that** the addition of the sub-micron-sized solid particles takes place by using a precipitation agent (12) which contains colloid particles.

11. A process according to one of the preceding claims, **characterised in that** the temperature control in the retention stretch is carried out such that a substantially constant supersaturation is present in the solution.

## Revendications

1. Procédé de production continue de substance médicamenteuse à inhaler comprenant les étapes suivantes :
- Utilisation d'une solution de substance médicamenteuse (11),
- Segmentation de la solution au moyen d'un segmenteur et d'un milieu de transport (22),
- Introduction et exécution du processus de cristallisation dans un parcours de rétention (3) au moyen de la gravure d'une température définie ; au cours duquel le processus de formation de germes cristallins est introduit d'abord au moyen d'une réduction par étape de la température, de façon à ce que la solution revête, selon la température T et la concentration C de la substance dissoute dans cette solution un état saturé, métastable ou instable puis que la croissance des cristaux soit influencée par un refroidissement ciblé, et
- Séparation des particules de cristaux des phases restantes à la fin du parcours de rétention dans un séparateur.

2. Procédé selon la revendication 1, **caractérisé en ce que** la solution de substance médicamenteuse (11) est mise à disposition par la dissolution de la substance médicamenteuse solide dans un solvant pour former une solution de substance médicamenteuse (11) de ce genre.

3. Procédé selon la revendication 1 ou 2, **caractérisé en ce que**
- la segmentation de la solution s'effectue au moyen d'un segmenteur et d'un milieu de transport (22) par formation de conditions piston.

4. Procédé selon l'une quelconque des revendications précédentes, **caractérisé en ce que**
- la solution de substance médicamenteuse (11) est mélangée avec un précipitant (12) dans un mélangeur pour former une solution précipitante homogène (21) et que cette solution précipitante passe par le procédé ultérieur.

5. Procédé selon l'une quelconque des revendications précédentes, **caractérisé en ce que** des particules solides sous-micrométriques sont introduites pour la formation de germes cristallins dans le segmenteur.

6. Procédé selon la revendication 5, **caractérisé en ce que** l'introduction de particules solides sous-micrométriques s'effectue lors de l'utilisation d'air comme milieu de transport (22) par l'adjonction de la substance médicamenteuse comme aérosol de pulvérisation ou par l'adjonction d'une substance secondaire comme aérosol de pulvérisation dans le flux d'air.

7. Procédé selon la revendication 5, **caractérisé en ce que**, lors de l'utilisation d'un milieu de transport (22) liquide des particules solides sous-micrométriques sont ajoutées sous forme de particules secondaires ou de particules de substance médicamenteuse au milieu de transport.

8. Procédé selon la revendication 5, **caractérisé en ce que** lors de l'utilisation d'un milieu de transport (22) liquide des particules solides sous-micrométriques sont ajoutées sous forme de colloïdes secondaires au milieu de transport.

9. Procédé selon la revendication 4, **caractérisé en ce que** des particules solides sous-micrométriques sont ajoutées dans le segmenteur pour la formation de germes cristallins.

10. Procédé selon la revendication 9, **caractérisé en ce que** l'adjonction des particules solides sous-micrométriques s'effectue en utilisant un précipitant (12), lequel contient des particules colloïdes.

11. Procédé selon l'une quelconque des revendications précédentes, **caractérisé en ce que** le maintien de la température s'effectue dans le parcours de rétention de façon à ce qu'il y ait une saturation essentiellement constante dans la solution.
